# EUROPEAN PATENT APPLICATION

(11) **EP 1 693 002 A1**
(43) Date of publication of application: **23.08.2006**
(21) Application number: 05003595.5
(22) Date of filing: 18.02.2005
(51) Int. Cl.: A61B 5/04

(54) **Device for measuring electrocardiogram with tapeless format and its method**

(71) Applicant: Lin, Kang-Ping, Jhongli City Taoyuan County Taiwan (TW)
(72) Inventor: Lin, Kang-Ping, Jhongli City Taoyuan County Taiwan (TW); Hsieh, Chih-Hui, Jhudong Town Hsinchu County Taiwan (TW)
(74) Representative: Beck, Michael Rudolf

(57) **Abstract**

A device for measuring electrocardiogram with tapeless format. The device measures electrocardiogram signals via electrodes with flat shape touching with two roots between total four fingers of two hands or electrodes embedded-in thereon only touching with two fingers for two hands. The device can display the electrocardiogram measured directly without adding any conductive material. The device comprises a shell, shaped as a thin and long cube and having at least one operating panel; two gelless electrodes with thin foil shape, slightly embedded and fixed in the operating panel and extended and surrounded at least one edge of the shell to a surface opposite to the operating panel; at least one information display, located on the operating panel to display a plurality of measured values; a calculation system, connecting with two gelless electrodes and the information display located in the shell in order to calculate relative electrical information measured from the gelless electrodes and display results on the information display.

## Description

### DESCRIPTION OF THE INVENTION

### Field of the Invention

The present invention is in relative to a device for measuring electrocardiogram with tapeless format and its method, especially the electrocardiogram measure device which measures electrocardiogram signals via electrodes with flat shape touching with two roots between total four fingers for two hands or electrodes embedded-in thereon only touching with two fingers for two hands, the invention can display the electrocardiogram measured directly without adding any electric conductive material.

### Background of the Invention

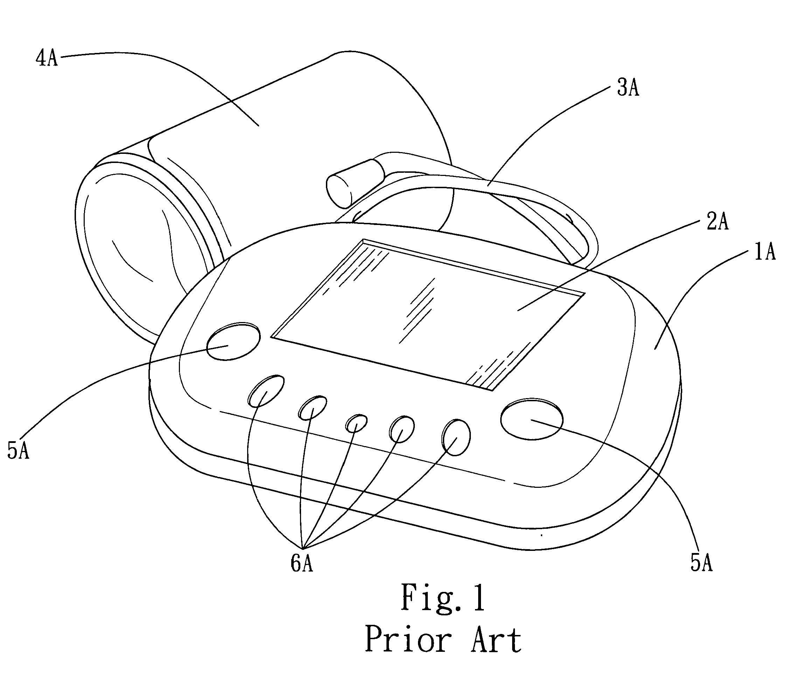

With more and more convenient in our daily life, human being lack of the exercise what they should have, therefore all kinds of modem diseases are happened. Due to the increment of the situations mentioned above, fitness clubs or gymnasia are established rapidly to provide exercise space for people living in the city to improve their health; secondly, personal medical instruments are produced, wherein the instruments to detect hearts blood vessels are the most.
Traditionally, two wrists, two ankles and some surface of patient body must be pasted a layer of conductive material or electrode pieces while having electrocardiography in hospital. If the patient have the symptom with fever caused by sickness, the patient would be uncomfortable while taking off sleeves, pants, smearing over a layer of sticky conductive material and furthermore some electrode pieces clamping and adhering to two wrists, two ankles and the chest. Hence, Taiwan Patent Number 503735 shows the latest product, please refer to Fig. 1, which is a prior art of the present invention. The prior art comprises a shell 1A with a display 2A two electrode touching keys 5A plural function keys 6A, a pipe line 3A connect to a wrist air bag 4A. Understood from Fig. 1, the wrist air bag 4A wraps around a wrist and then the two electrode touching keys 5A are filled out with conductive, continuously two finger tips touch the two electrode touching keys 5A for grabbing relative electric signals about a heart blood vessels so as to present results on the display 2A after calculation. However, this prior art still needs to add conductive liquid to electrode touching keys 5A when collecting relative information. An additional can loaded with conductive liquid shall occupy some space for storing, and it would be inconvenient to carry; on the other hand, as aforesaid, the way of adding conductive liquid may be improved and avoided.

### SUMMARY OF THE INVENTION

The present invention is a device for measuring electrocardiogram with tapeless format and its method to solve the inconvenience and uncomfortable feeling caused by the way of adhering electrode pieces and pasting conductive liquid, especially the present invention only needs two metal electrodes with thin shapes clamped by two roots of four fingers of a right and a left hands or electrodes embedded-in thereon only touching with two fingers for two hands without pasting any conductive liquid to obtain correlative information of the electrocardiogram.
The present invention comprises a shell, shaped as a thin and long cube and having at least one operating panel, the operating panel further comprising at least one button for setting and transferring functions; two gelless electrodes with thin foil shape, slightly embedded and fixed in the operating panel and extended and surrounded at least one edge of the shell to a surface opposite to the operating panel; at least one information display, located on the operating panel to display a plurality of measured values; a calculation system, connecting with two gelless electrodes and the information display located in the shell in order to calculate relative electrical information measured from the gelless electrodes and display results on the information display. It is to be understood that both the foregoing general description and the following detailed description are exemplary and explanatory only and are not restrictive of the invention, as claimed.
The accompanying drawing, which is incorporated in and constitutes a part of this specification, illustrates several embodiments of the invention and together with the description, serves to explain the principles of the invention.

### BRIEF DESCRIPTION OF THE DRAWINGS

Fig. 1, which is a prior art of the present invention;
Fig. 2 is a 3-D sketch of a front view of a preferred embodiment of the present invention;
Fig. 3 is a 3-D sketch of a rear view of the preferred embodiment of the present invention;
Fig. 4 is a structural sketch of hardware of the calculation system of the present invention;
Fig. 5 is a flowchart of measuring steps of the present invention;
Fig. 6 is a 3-D sketch of a front view of a second preferred embodiment of the present invention;
Fig. 7 is a 3-D sketch of a rear view of the second preferred embodiment of the present invention;
Fig. 8 is a flowchart of measuring steps of the second preferred embodiment of the present invention;
Fig. 9 is a 3-D sketch of a front view of a third preferred embodiment of the present invention;
Fig. 10 is a flowchart of measuring steps of the third preferred embodiment of the present invention.

### DESCRIPTION OF THE EMBODIMENTS

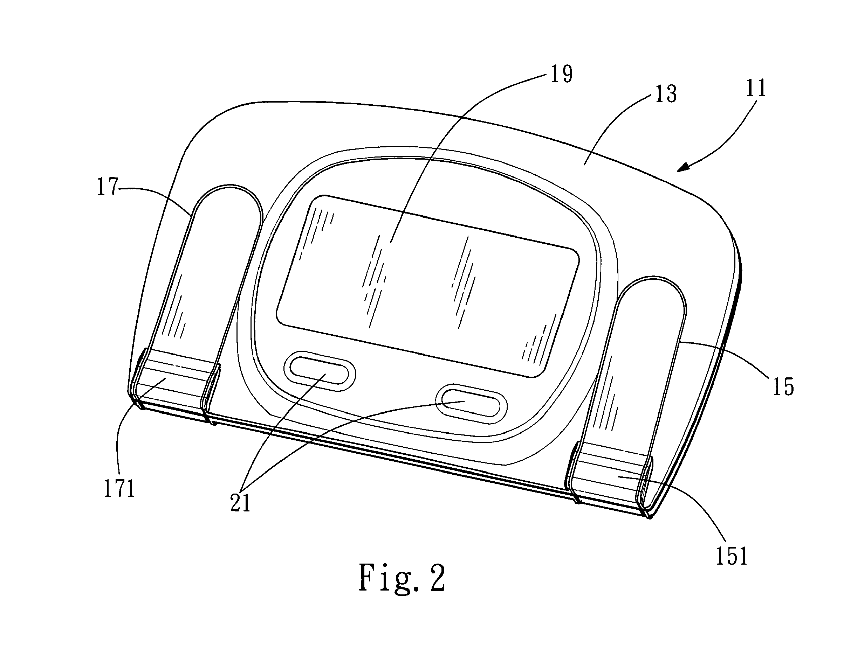

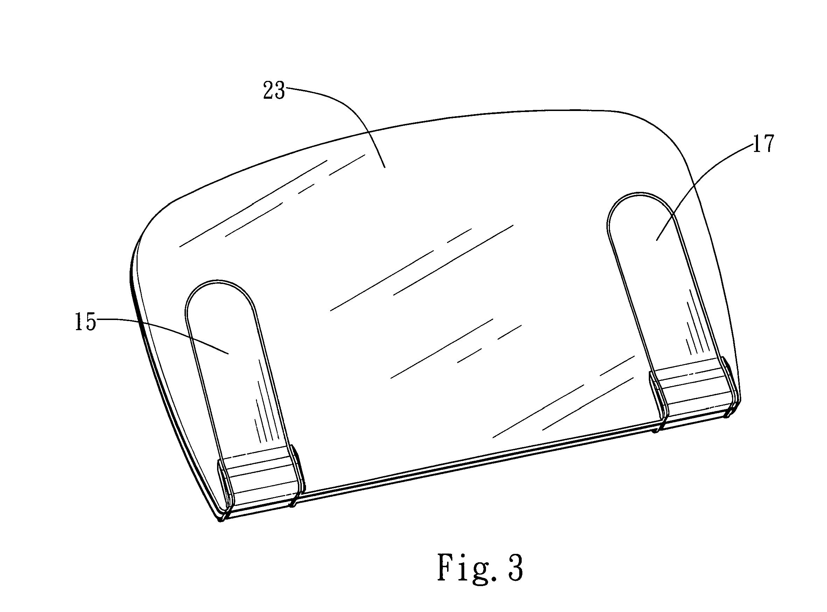

Please refer to Fig. 2 and Fig. 3, which are a 3-D sketch of a front view and a 3-D sketch of a rear view of the preferred embodiment of the present invent. The present invention comprises a shell 11, shaped as a thin and long cube and having an operating panel 13, the operating panel 13 further comprising two buttons 21 for setting and transferring functions; two gelless electrodes with thin foil shape, a right and a left electrodes 15, 17, slightly embedded and fixed in the operating panel 13 and extended and surrounded an edge of the shell 11 to a bottom surface 23 opposite to the operating panel 13, electrodes 15 and 17 being made by any conductive metal or conductive rubber, further, both places of electrodes 15 and 17 surrounding the edge of the shell 11 individually having protruding surfaces or ridges 151 and 171 for helping two roots of four fingers of two hands to clamp closely; an information display 19, located on the operating panel 13 to display a plurality of measured values; a calculation system 27 (not shown on Fig. 2 and Fig. 3), connecting with electrodes 15 and 17 and the information display 19 located in the shell 11 in order to calculate relative electrical information measured from the electrodes 15 and 17 and displaying the measured values on the information display 19.

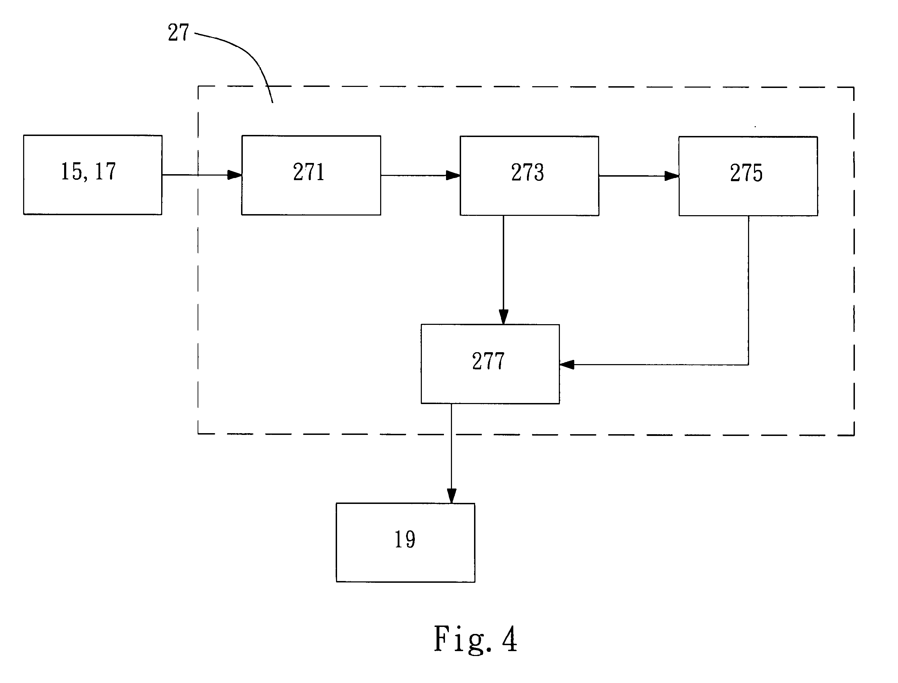

Please refer to Fig. 4, which is a structural sketch of hardware of the calculation system of the present invention. Fig. 4 discloses a relationship among all main components in the calculation system 27 and another relationship among the calculation system 27, electrodes 15 and 17 and information display 19. The calculation system 27 comprises a pre-signal amplify circuit 271, an electrocardio signal amplify/filter circuit 273, an analog/digital converting circuit 275 and a CPU 277. Arrows shown in Fig. 4 are the process for calculation, it means the pre-signal amplify circuit 271 amplifies signals of received electrical information and constantly the electrocardio signal amplify/filter circuit 273, the analog/digital converter circuit 275 and the CPU 277 execute calculation when right and leftelectrodes 15 · 17 receiving relative electrical information. Wherein, a loop is formed by electrocardio signal amplify/filter circuit 273, analog/digital converter circuit 275 and CPU 277, whose main function is to process different information values including ST segment QRS interval and heartbeat rate. At last, these information values are shown on the display 19.

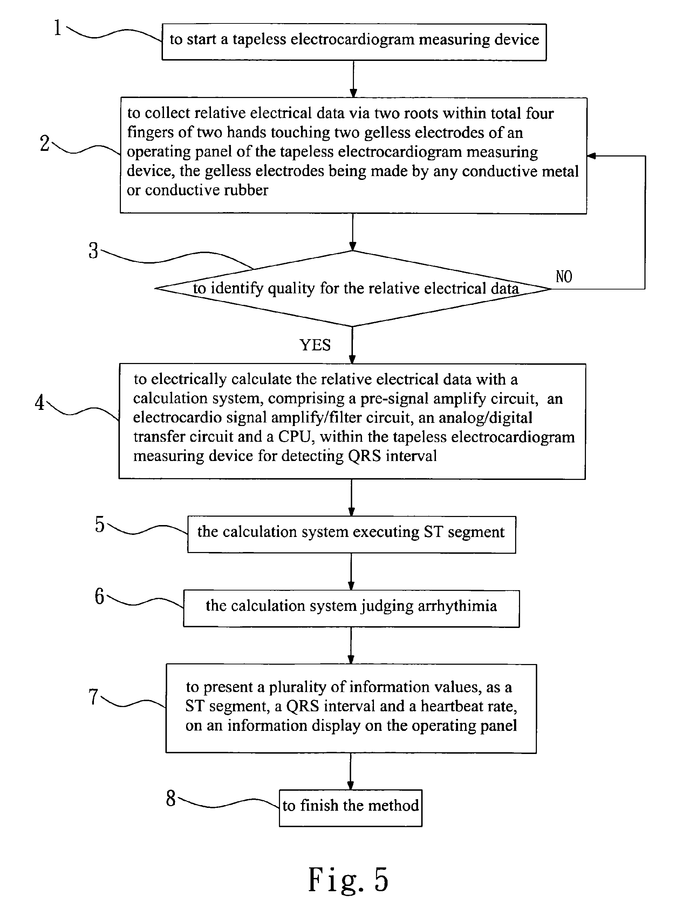

Please refer to Fig. 5, which is a flowchart of measuring steps of the present invention. The steps include:
(1) to start a tapeless electrocardiogram measuring device;
(2) to collect relative electrical data via two roots within total four fingers of two hands touching two gelless electrodes of an operating panel of the tapeless electrocardiogram measuring device, the gelless electrodes being made by any conductive metal or conductive rubber;
(3) to identify quality for the relative electrical data, if unacceptable, then returning to step (2), otherwise going to next step;
(4) to electrically calculate the relative electrical data with a calculation system, comprising a pre-signal amplify circuit, an electrocardio signal amplify/filter circuit, an analog/digital transfer circuit and a CPU, within the tapeless electrocardiogram measuring device for detecting QRS interval;
(5) the calculation system executing ST segment;
(6) the calculation system judging arrhythimia;
(7) to present a plurality of information values, as a ST segment, a QRS interval and a heartbeat rate, on an information display on the operating panel;
(8) to finish the method.

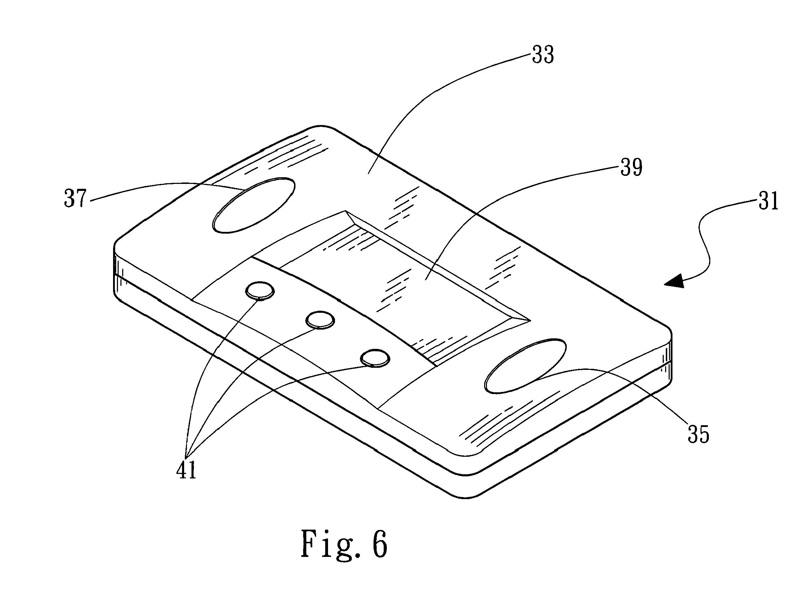

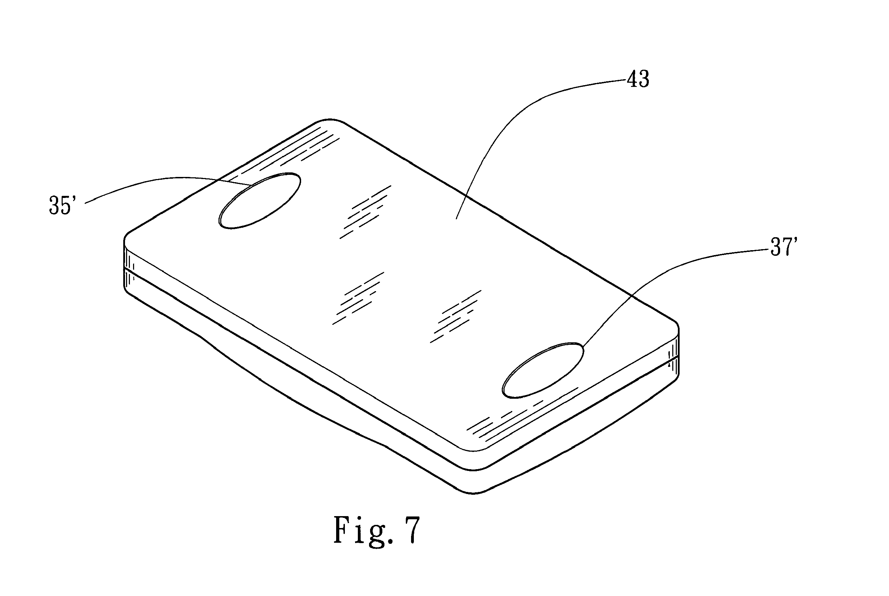

Please refer to Fig. 6 and Fig. 7, which are a 3-D sketch of a front view of a second preferred embodiment of the present invent and a 3-D sketch of a rear view of the second preferred embodiment of the present invent. The device for measuring electrocardiogram with tapeless format comprises: a shell 31, shaped as a thin and long cube and having one operating panel 33, the operating panel 33 including three buttons 41 to set and transfer functions; four gelless electrodes, two right electrodes 35, 35' and two left electrodes 37, 37' , slightly embedded and fixed in the operating panel 33 and a bottom surface 43, the gelless electrodes being made by any conductive metal or conductive rubber; one information display 39, located on the operating panel 33 to display a plurality of measured values, the plurality of information values shown on the information display 39 including values of ST segment, QRS interval and heart-beat rate; a calculation system (not shown in Fig. 6 and Fig. 7, referring to Fig. 4), connecting with the four gelless electrodes 35, 35', 37 and 37' and the information display 39 located in the shell 31 in order to calculate relative electrical information measured from the gelless electrodes 35, 35', 37 and 37' and display results on the information display 39, the calculation system further comprising a pre-signal amplify circuit, an electrocardio signal amplify/filter circuit, an analog/digital transfer circuit and a CPU, wherein the pre-signal amplify circuit connected to the gelless electrodes to get relative electrical data, and continuously results being displayed on the information display 39 after calculating the electrical data by means of the electrocardio signal amplify/filter circuit and the analog/digital transfer circuit and the CPU.

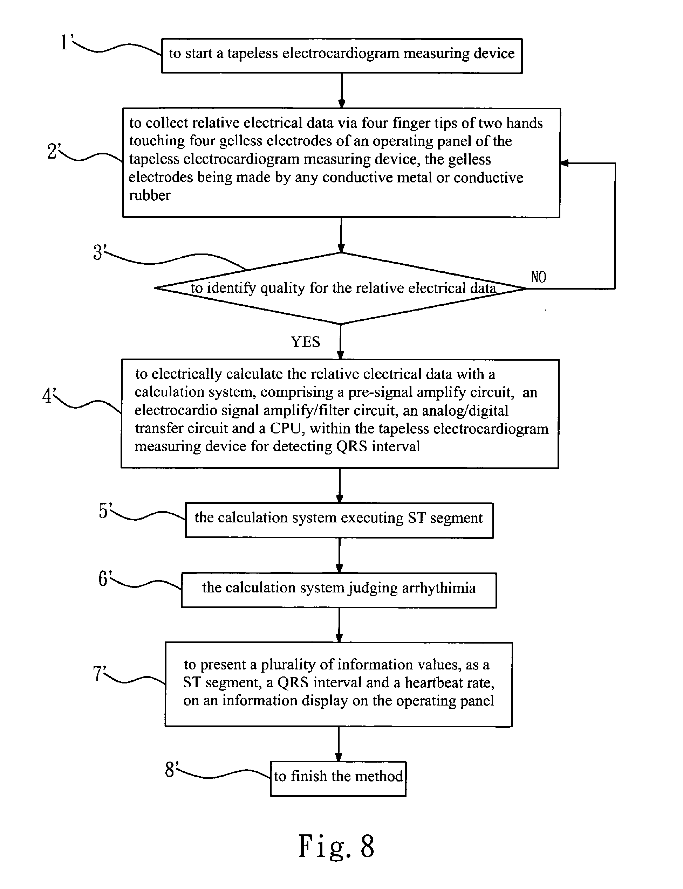

Please refer to Fig. 8, which is a flowchart of measuring steps of the second preferred embodiment of the present invention. The steps include:
(1') to start a tapeless electrocardiogram measuring device;
(2') to collect relative electrical data via four finger tips of two hands touching four gelless electrodes of an operating panel of the tapeless electrocardiogram measuring device, the gelless electrodes being made by any conductive metal or conductive rubber;
(3') to identify quality for the relative electrical data, if unacceptable, then returning to step (2), otherwise going to next step;
(4') to electrically calculate the relative electrical data with a calculation system, comprising a pre-signal amplify circuit, an electrocardio signal amplify/filter circuit, an analog/digital transfer circuit and a CPU, within the tapeless electrocardiogram measuring device for detecting QRS interval;
(5') the calculation system executing ST segment;
(6') the calculation system judging arrhythimia;
(7') to present a plurality of information values, as a ST segment, a QRS interval and a heartbeat rate, on an information display on the operating panel;
(8') to finish the method.

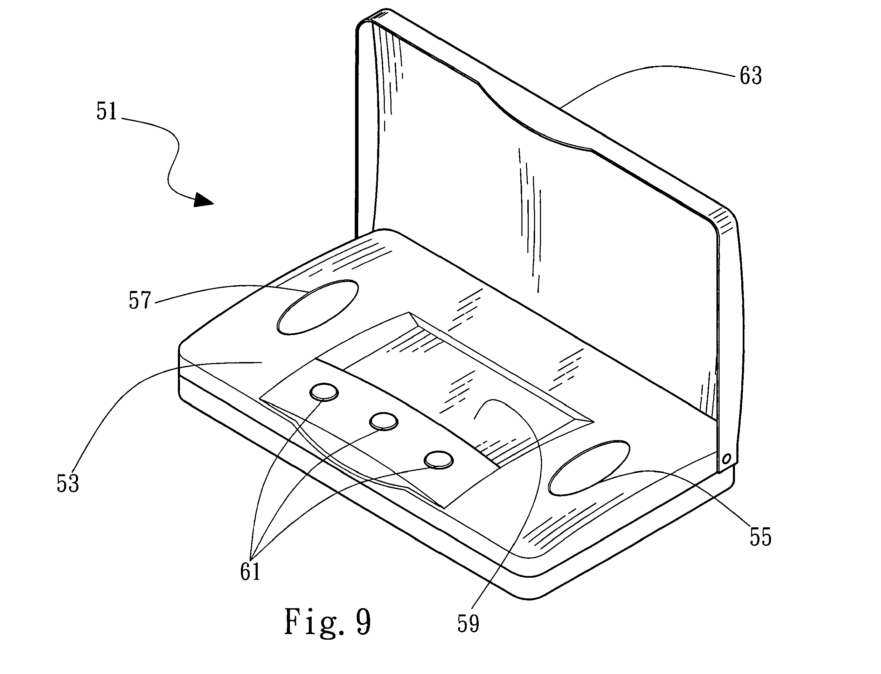

Please refer to Fig. 9, which is a 3-D sketch of a front view of a third preferred embodiment of the present invent. The device for measuring electrocardiogram with tapeless format comprises: a cover 63; a shell 51, shaped as a thin and long cube and having one operating panel 53, the operating panel 53 including three buttons 61 to set and transfer functions; two gelless electrodes 55 and 57, slightly embedded and fixed in the operating panel 53, the gelless electrodes being made by any conductive metal or conductive rubber; one information display 59, located on the operating panel 53 to display a plurality of measured values, the plurality of information values shown on the information display 59 including values of ST segment, QRS interval and heart-beat rate; a calculation system (not shown in Fig. 9, referring to Fig. 4), connecting with the two gelless electrodes 55 and 57 and the information display 59 located in the shell 51 in order to calculate relative electrical information measured from the gelless electrodes 55 and 57 and display results on the information display 59, the calculation system further comprising a pre-signal amplify circuit, an electrocardio signal amplify/filter circuit, an analog/digital transfer circuit and a CPU, wherein the pre-signal amplify circuit connected to the gelless electrodes to get relative electrical data, and continuously results being displayed on the information display 59 after calculating the electrical data by means of the electrocardio signal amplify/filter circuit and the analog/digital transfer circuit and the CPU.

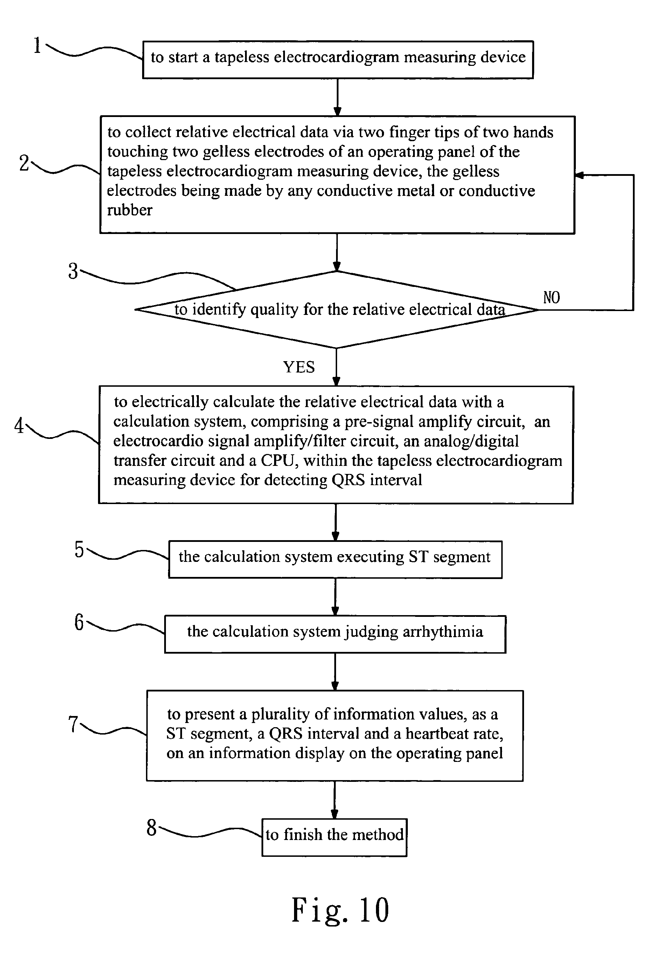

Please refer to Fig. 10, which is a flowchart of measuring steps of the third preferred embodiment of the present invention. The steps include:
(1") to start a tapeless electrocardiogram measuring device;
(2") to collect relative electrical data via two finger tips of two hands touching two gelless electrodes of an operating panel of the tapeless electrocardiogram measuring device, the gelless electrodes being made by any conductive metal or conductive rubber;
(3") to identify quality for the relative electrical data, if unacceptable, then returning to step (2), otherwise going to next step;
(4") to electrically calculate the relative electrical data with a calculation system, comprising a pre-signal amplify circuit, an electrocardio signal amplify/filter circuit, an analog/digital transfer circuit and a CPU, within the tapeless electrocardiogram measuring device for detecting QRS interval;
(5") the calculation system executing ST segment;
(6") the calculation system judging arrhythimia;
(7") to present a plurality of information values, as a ST segment, a QRS interval and a heartbeat rate, on an information display on the operating panel;
(8") to finish the method.

The device for measuring electrocardiogram with tapeless format as described above can be applied on other electrical products, which can be one of the following: mobile phone, walkie-talkie, portable computer, walkman and the relatives. The main purpose is to let people understand their health right away, therefore any disease for heart or blood vessel can be avoided in advance.

Known from above description, the present invention adopts a unique measuring method completely different than prior arts to collect the relative electrical data of physiology by touching two thin electrodes with the roots of total four fingers of two hands or electrodes embedded in thereon only touching with two fingers for two hands. Wherein, there is only two thin electrodes without smearing any conductive material on skin or electrodes, and the present invention can be instead of the complex measuring process with pasting the electrodes on hand ankle and chest; on the other hand, the measuring apparatus for the present invention can be purified and lighter due to easier measuring method.

It is to be understood that while the invention has been described above in conjunction with preferred specific embodiments, the description and examples are intended to illustrate and not limit the scope of the invention, which is defined by the scope of the appended claims.

## Claims

1. Device for measuring electrocardiogram with tapeless format comprising:
a shell, shaped as a thin and long cube and having at least one operating panel;
two gelless electrodes, slightly embedded and fixed in the operating panel;
at least one information display, located on the operating panel to display a plurality of measured values;
a calculation system, connecting with two gelless electrodes and the information display located in the shell in order to calculate relative electrical information measured from the gelless electrodes and display results on the information display.

2. Device for measuring electrocardiogram with tapeless format as described in claim 1, wherein the two gelless electrodes are with thin foil shape, slightly embedded and fixed in the operating panel and extended and surrounded at least one edge of the shell to a surface opposite to the operating panel;

3. Device for measuring electrocardiogram with tapeless format as described in claim 1 or 2, wherein the operating panel has at least one button to set and transfer functions.

4. Device for measuring electrocardiogram with tapeless format as described in one of claims 1 to 3, wherein the gelless electrodes can be made by any conductive metal.

5. Device for measuring electrocardiogram with tapeless format as described in one of claims 1 to 4, wherein a plurality of information values shown on the information display include but not limit to the values of ST segment, QRS interval and heart-beat rate.

6. Device for measuring electrocardiogram with tapeless format as described one of claims 1 to 5, wherein the calculation system further comprises:
a pre-signal amplify circuit;
an electrocardio signal amplify/filter circuit;
an analog/digital transfer circuit;
a CPU;
wherein the pre-signal amplify circuit is connected to the gelless electrodes to obtain relative electrical data, and continuously displays results on the information display after calculating the electrical data by means of the electrocardio signal amplify/filter circuit and the analog/digital transfer circuit and the CPU.

7. Device for measuring electrocardiogram with tapeless format as described in one of claims 1 to 6, wherein the device further comprises another two gelless electrodes, slightly embedded and fixed in another surface opposite to the operating panel.

8. Device for measuring electrocardiogram with tapeless format as described in one of claims 1 to 7, wherein the device can be applied on other electrical products, which can be one of the following: mobile phone, walkie-talkie, portable computer, walkman.

9. Method for measuring electrocardiogram with tapeless format comprising:
to start a tapeless electrocardiogram measuring device;
to collect relative electrical data via at least one finger tip from each hand touching two gelless electrodes of an operating panel of the tapeless electrocardiogram measuring device;
to identify quality for the relative electrical data, if unacceptable, then returning to step (2), otherwise going to next step;
to electrically calculate the relative electrical data with a calculation system within the tapeless electrocardiogram measuring device;
to present a plurality of information values on an information display on the operating panel;
to finish the method.

10. Method for measuring electrocardiogram with tapeless format as described in claim 9, wherein the gelless electrodes can be made by any conductive metal.

11. Method for measuring electrocardiogram with tapeless format as described in claim 9 or 10, wherein the calculation system further comprises:
a pre-signal amplify circuit;
an electrocardio signal amplify/filter circuit;
an analog/digital transfer circuit;
a CPU.

12. Method for measuring electrocardiogram with tapeless format as described in one of claims 9 to 11, wherein the electrical calculation by order in step (4) is detection for QRS, detection for ST and judgment of cardiac arrhythmia.

13. Method for measuring electrocardiogram with tapeless format as described in one of claims 9 to 12, wherein the information values displayed on the information display include at least ST segment, QRS interval and heartbeat rate.
